# EUROPEAN PATENT SPECIFICATION

(11) **EP 1 480 565 B1**
(45) Date of publication and mention of the grant of the patent: **10.12.2008**
(21) Application number: 03716279.9
(22) Date of filing: 03.03.2003
(51) Int. Cl.: A61B 17/00

(54) **VASCULAR OCCLUSION DEVICE**
VASKULÄRE OKKLUSIONSVORRICHTUNG
DISPOSITIF D'OCCLUSION VASCULAIRE

(30) Priority: 01.03.2002 US 361122 P
(43) Date of publication of application: 01.12.2004
(73) Proprietor: REGENTS OF THE UNIVERSITY OF MINNESOTA, Minneapolis, MN 55455 (US); DAS, Gladwin S., Arden Hills, MN 55112 (US)
(72) Inventor: DAS, Gladwin, S., Arden Hills, MN 55112 (US)
(74) Representative: Preuss, Udo
(86) International application number: PCT/US2003/006473
(87) International publication number: WO 2003/073944

(56) References cited:
- WO-A-01/17435
- WO-A-96/32882
- GB-A- 2 269 321
- US-A- 5 312 435
- US-A- 5 342 393
- US-A- 5 350 399
- US-A- 5 827 325
- US-A- 5 904 703
- US-B1- 6 312 446

## Description

### Field of the Invention

This invention relates to the field of medical devices, and more specifically to an apparatus for vascular occlusion.

### Background

Vascular occlusion devices such as atrial septal defect closure devices are used for closing defects or holes in the vascular system of a body. Such devices usually include an occluding member which is dimensioned larger than the hole, and some technique for mounting the occluding member to the tissue proximate the defect or hole.

U.S. Patent No. 6,312,446 B1 (Buscemi Paul J. et al.*)* discloses a catheter delivered device to close a septal defect, the device comprising a cylindrical shaft of metal or polymeric material with concentric parallel cuts through the wall of the device which create flattened support struts. The center of the support struts move radially away from the axis in a hinge like fashion in response to the movement of the device's proximal and distal ends toward the center of the device. This movement is reversibly effected through mechanical means. The device can be coated with growth factors, mitogenic factors or other determinants which can improve tissue growth such that tissue ingrowth can occur over a period of time. The catheter itself may be an ultrasonic imaging catheter.

WO 96/32882 A (Heartport Inc.) discloses devices, systems, and methods for accessing the interior of the heart, and performing procedures therein while the heart is beating. A tubular access device is positioned through an intercostal space and through a muscular wall of the heart. The access device includes a balloon or flange for sealing the penetration to prevent leakage of blood. An obturator is positionable in the access device, and includes cutting means located at its distal end. Elongated instruments are introduced through the access device to perform surgical procedures including septal device repair, ablation, and electrophysiological mapping.

U.S. Patent No. 5,904,703 (Gibson Paul) discloses a transcatheter occluder device for closing an opening in a tissue wall that includes a body member formed of an open cell polymer foam capable of holding a predetermined occluder shape and being resiliently compressible for transcatheter deployment. The occluder device includes a pair of spaced-apart disks interconnected by a narrow neck portion. Grip means are provided in the neck portion to releasably engage a guide wire for deployment of the occluder device through a catheter.

WO 01/17435 A (Gainor et al.) discloses a septal defect closure device having a first occluding disk having a first flexible membrane attached to a first frame and a second occluding disk having a second flexible membrane attached to a separate second frame. The first frame has at least two outwardly extending loops joined to one another by flexible joints. These loops are attached to the first membrane to define taut fabric petals when the first disk is in a deployed configuration. A central portion of the first membrane is joined to the central portion of the second membrane via a joining segment, which may comprise a conjoint disk. The flexible joints of the first frame are received within the joining segment. This septal defect closure device provides enhanced retrievability.

GB Patent No. 2,269,321 A (Nat et al.) discloses an occluder device for a body duct that comprises first and second expansion assemblies connected to each other and provided with attachment means for connection to a wire and catheter positioning system, each assembly comprising a web of material and at least three resilient arms projecting, when undeformed, radially from the device and carrying the web, the arms being resiliently biased apart to open up and resiliently deformable to close down the associated web in an umbrella-like manner. The assemblies are joined at their central portions and arranged oppositely to each other. Each arm is bent through an angle such that, when the webs are opened up, the distal ends of the arms of each web point generally towards the other web and the two webs are biased into contact with one another at their peripheries.

U.S. Patent No. 5,350,399 (Erlenbacher et al.) recites that the sealing device is composed of an intra-arterial occluder and an extra-arterial occluder, both made of resilient biocompatible and/or bioabsorbable material and held in place via a saw-toothed guide extending integrally from the intra-arterial occluder. An insertion tool is provided to effect sliding of the extra-arterial occluder over the guide and fixation of the extra-arterial occluder to the guide in sealed relation over an opening in body tissue and safe cutting of the guide. A force gauge is provided on the tool to indicate the sealing pressure on the intra-arterial occluder.

U.S. Patent No. 5,342,393 (Stack Richard) recites that a method and device are provided for sealing a perforation in the wall of, for example, a blood vessel. The device is in the form of a two part closure which seals the hole by clamping the tissue surrounding the hole from both the inside and the outside of the vessel. In accordance with one exemplary embodiment, two rivet portions are provided which are bayonet or screw threadedly interlocked to clamp the vessel wall about the perforation. An alternate embodiment applies heat to couple the rivet parts as well as to cauterize the wound site. Heat may also advantageously be used to separate the rivet from its delivery system.

U.S. Patent No. 5,827,325 (Landgrebe et al.) recites an implant, in particular for the closure of trocar puncture points, that has a flexible, flat base part and a stopper part that starts from one side of the base part and extends in an essentially perpendicular manner relative to the base part. The stopper part can be provided with a waist in the vicinity of the base part.

The devices are typically made of a wire frame or skeleton which can become fatigued or perforate the tissue wall. It is also desirable to center the device within the hole and provide a stable technique for keeping the device securely mounted to the tissue.

### Brief Description of the Drawings

Figure 1A shows a front view of a vascular occlusion device according to one embodiment.
Figure 1B shows a perspective view of the vascular occlusion device of Figure 1A.
Figure 1C shows a side view of the vascular occlusion device of Figure 1A with the flanges folded outward.
Figure 1D shows side view of the vascular occlusion device of Figure 1A.
Figure 1E shows a side section view along line E-E of Figure 1B.
Figure 2 shows a detail of area 2 of Figure 1B.
Figure 3 shows a front perspective view of the vascular occlusion device of Figure 1A.
Figure 4 shows a side view of the vascular occlusion device of Figure 1A.
Figure 5 shows a detail of a front portion of the vascular occlusion device of Figure 1A.
Figure 6 shows a detail of a front portion of the vascular occlusion device of Figure 1A.
Figure 7 shows a rear perspective view of the vascular occlusion device of Figure 1A.
Figure 8A shows a front view of a vascular occlusion device according to one embodiment not forming part of the present invention.
Figure 8B shows a side sectional view along line B-B of the vascular occlusion device of Figure 8A.
Figure 8C shows a sectional view along line C-C of Figure 8A.
Figure 8D shows a view of the vascular occlusion device of Figure 8A.
Figure 8E shows a front perspective view of the vascular occlusion device of Figure 8A.
Figure 9 shows a front view of the vascular occlusion device of Figure 8A.
Figure 10 shows a rear perspective view of the vascular occlusion device of Figure 8A.
Figures 11A-11C show side, top and bottom view of a device according to one embodiment not forming part of the present invention.
Figure 12A shows a device in accordance with one embodiment.
Figure 12B shows a top view of a portion of the device of Figure 12A.
Figure 12C shows a cross section of line C-C of Figure 12B.
Figure 12D shows a cross-section of line D-D of Figure 12B.
Figures 13A-13D show the device of Figure 12A being deployed.

### Detailed Description

In the following detailed description, reference is made to the accompanying drawings which form a part hereof, and in which is shown exemplary embodiments of the invention.

These embodiments are described in sufficient detail to enable those skilled in the art to practice the invention, and it is to be understood that other embodiments may be utilized and that structural changes may be made without departing from the scope of the present invention. Therefore, the following detailed description is not to be taken in a limiting sense, and the scope of the present invention is defined by the appended claims.

The present system provides a device that can be used to occlude defects in the vascular system by minimally invasive means. Patients are often born with congenital defects in the heart commonly alluded to as "holes in the heart." This may be between the upper chambers in the heart called "atrial septal defects" or between the lower chambers of the heart called" ventricular septal defects. "In other instances, it may be "patent ductus arteriosii" when it is a communication between the aorta and pulmonary artery. In addition, there may be arteriovenous fistulae at several sites within the body, or the presence of collateral arteries to the lungs that bring excessive amounts of blood and require closure.

In recent years, after diagnostic or therapeutic cardiac catheterization, it has become possible to close the arterial puncture site with devices, and because of the anticoagulation they receive, the potential for bleeding and prolonged bed rest can be avoided. However the devices for the closure of heart defects or femoral hemostatic devices have clinical problems and there is a need for a more optimal method of closure.

In the case of cardiac septal defects, some present devices are made of metallic frameworks with single or multiple metallic wires in different geometries. These have the potential of erosion into surrounding structures and corrosion as these implants in children will remain in place for 70 years and more. Some self-centering devices have decreased the potential for residual leaks and permit the almost complete occlusion of these defects. However some of these devices are bulky, and made of several bulky wires.

In some embodiments, the present system includes a vascular occlusion device that can be self-centering, can completely occlude defects, and can also be retrieved back into the delivery catheter in case of sub-optimal deployments.

In one embodiment, a vascular occlusion device can include no metallic components. For example, the device can be made from bio-compatible plastic materials that permit it to be injection molded, vacuum formed, or extruded.

This makes the manufacturability of such a device quite simple and large quantities can be manufactured quite easily. In one embodiment, the device is made from a bio-adsorbable polymer or a bio-degradable polymer. After deployment of the device, such a polymer is gradually replaced by fibrous tissue and resorbed over a period of months. No metallic components remain and this permits the most desirable form of repair of such defects. In other instances, where a bio-adsorbable material is not appropriate, for fear of aneurysm formation in high pressure shunts, silicone elastomers, polyurethanes or combinations of the two can be used.

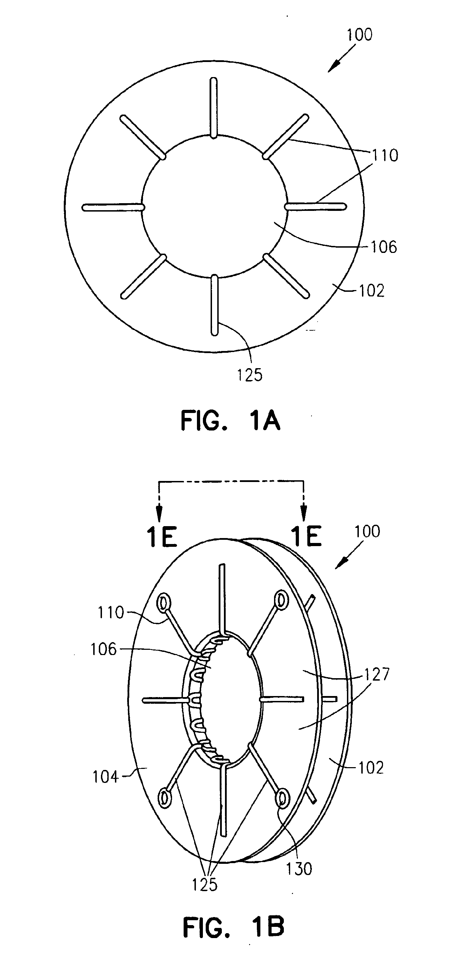

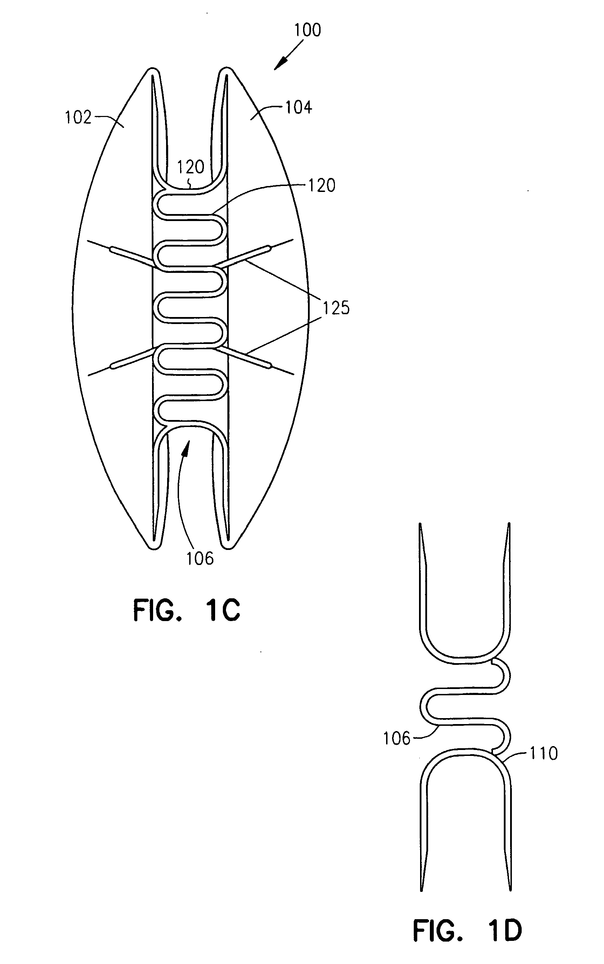

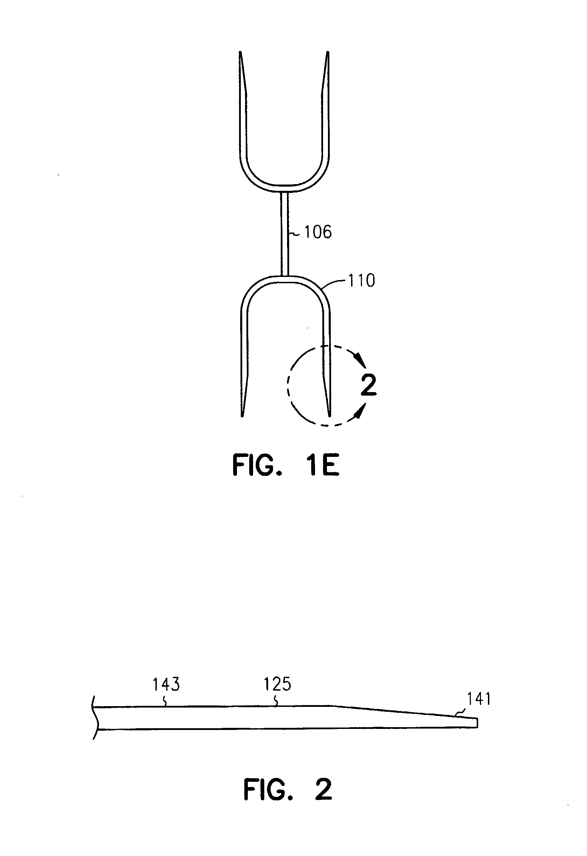

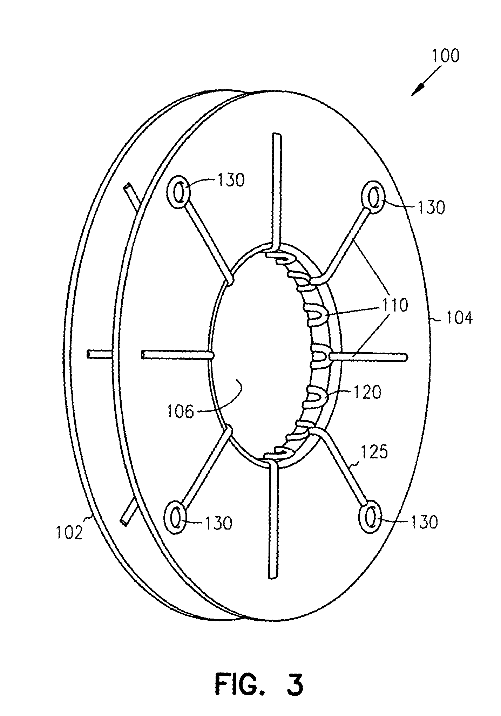

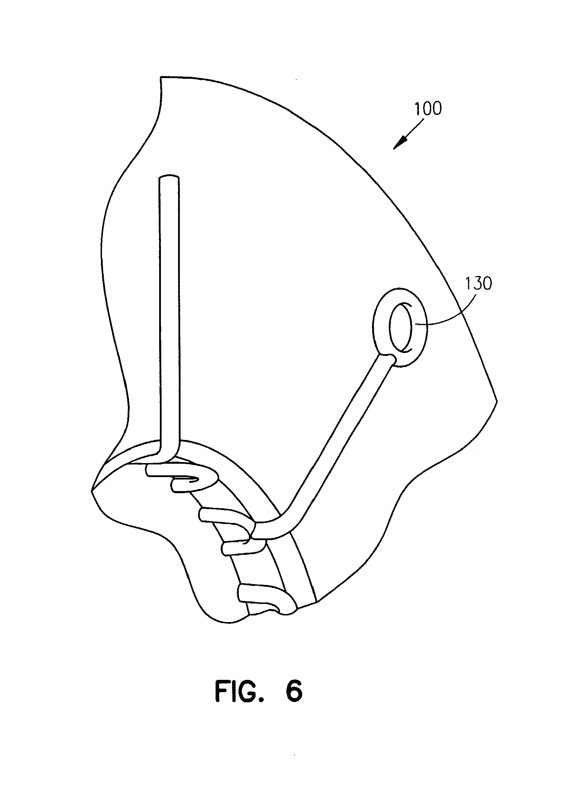

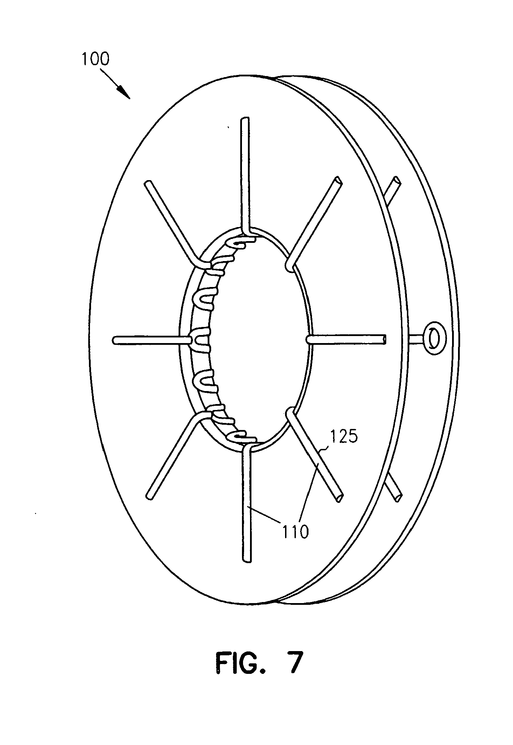

Figure 1A-7 show a vascular occlusion device 100 according to one embodiment. Device 100 includes a double disk device, with two flanges 102 and 104 that extend outwards from a central area 106. In one embodiment, the central area 106 is dimensioned to act as a self-centering mechanism to allow the flanges and the device to be substantially centered over the defect. The central area occludes the defect and extends to the double flanges circumferentially. In one embodiment, central area 106 has a diameter of approximately 12 mm, with the flanges 102 and 104 having a diameter of approximately 30 mm.

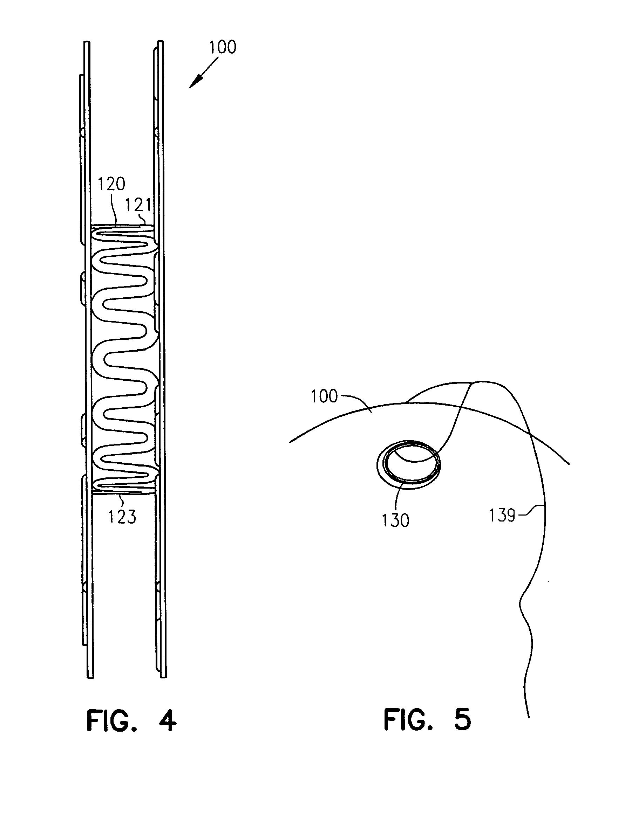

Device 100 includes a self-expanding skeleton structure 110 that provides the major structural strength of the device. Skeleton structure 110 includes a plurality of ribs 125. Between each of the ribs 125 are thinner membrane portions 127. In one example, the membrane portions 127 can be approximately 0,254 mm (0.01 inches) thick, or less. Ribs 125 can be approximately 0,635 mm (0.025 inches) thick. Skeleton 110 supports a main body structure which includes the flanges 102 and 104. As seen in Figures 1C, 1D, 3, 4, 5, 6 and 7, the skeleton has a sinuous structure around the central rim 120 that permits the rim area to be collapsed down. From this, there are the plurality of spoke-like projections or ribs 125 that permit folding of the flanges. In one example, ribs 125 extend radially along each disk. On one of the disks, one or more holes 130 are incorporated whose function is to be the sites to tether it to the release mechanism (see Figure 5) with a tether 139. In one embodiment, holes 130 are located at the ends of ribs 125 with a section of rib encircling the hole.

Device 100 is flexible, compressible, and self-expandable and can be used in a manner known in the art. In one example use, the device 100 can be collapsed into a catheter by folding the flanges 102 and 104 outwards and collapsing the central rim 120. The catheter is then passed through an atrial or ventricular septal defect, or other hole in a body. The first disk 102 of device 100 is pushed out of the catheter and springably opens on one side of the defect. The rib configuration of the skeleton structure of each flange automatically expands the flange to its original dimensions on release from the confines of the catheter. The expanded disk 102 is then drawn back to the defect. The catheter is pulled back, and second disk 104 then springably opens on the second side of the defect. The central rim area 120 also expands to its original size and abuts the inner diameter surface of the defect. This acts as a centering mechanism to center the flanges of the device on each side of the defect. Referring to Figure 4, the outer surface of rim 120 diametrically extends from a first surface 121 to a second surface 123. These surfaces and the rest of the expanded rim surface are biased towards and can contact the inner surface of the defect to help center the device.

Once deployment of the device is considered optimal, the device is released. If deployment is sub-optimal, tethers 139, which are attached at one or more of holes 130 can be used to retrieve the device. By pulling back on the tethers, the device can be re-collapsed into the catheter. Again, holes 130 are structurally connected to ribs 125. Thus, when the tethers are pulled back, the force against the ribs of the skeleton structure force flange 104 to collapse. The device can then be drawn back into the catheter to be re-deployed. Figure 2 shows one example of a distal end of a rib 125 of device 100. In this example, the end 141 of rib 125 is thinner (approximately 0,254 mm (0.01 inches)) than the inner portion 143 (approximately 0,635 mm (0.025 inches)) of the rib. This provides that the periphery area of each flange is a little more flexible and softer than the middle regions. This can help eliminate tissue erosion by the device once it is implanted. Figure 2 shows one example of a distal end of a rib 125 of device 100. In this example, the end 141 of rib 125 is thinner (approximately 0,254 mm (0.01 inches)) than the inner portion 143 (approximately 0,635 mm (0.025 inches)) of the rib. This provides that the periphery area of each flange is a little more flexible and softer than the middle regions. This can help eliminate tissue erosion by the device once it is implanted.

Again, in one embodiment device 100 is molded from a non-metallic material. Such an occlusion device has better tolerance than prior devices and has better long-term stability. Possible materials include: silicone elastomers, polyurethanes, or combinations of the two, and other compatible materials. In one example, device 100 is a one-piece, completely integrated device formed of a single material with ribs 110 being thicker parts of the material. This allows for fast manufacturability since the device can be molded or otherwise formed in a single process. In some embodiments, the ribs are formed of one type of material and the membrane portion from a different type of material.

In one embodiment, the device includes a bio-adsorbable material.

Possible bio-adsorbable material include: polyglycolic acid, polydioxanone, polylactic acid, and polycaprolactone, etc. Some embodiments use other bio-degradable and bioabsorbable plastics. Again, in some embodiments, these poly materials can be used to form the device as a single integrated unit formed of a single, moldable material. Using a bioabsorbable material as described above allows the device to slowly be resorbed into the body while the poly material is replaced by tissue. This results in a closed defect and leaves no metallic parts in a body.

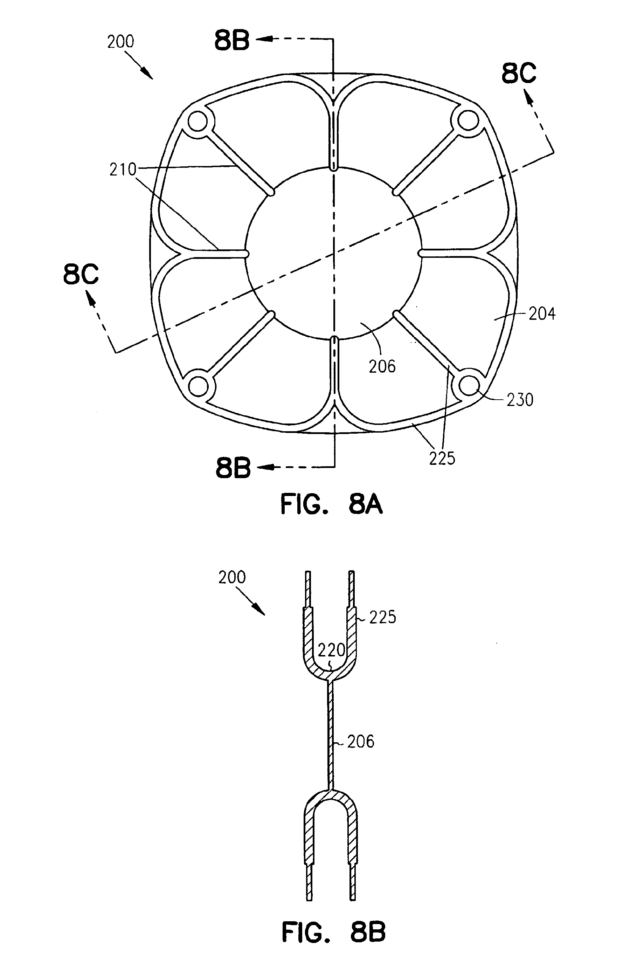

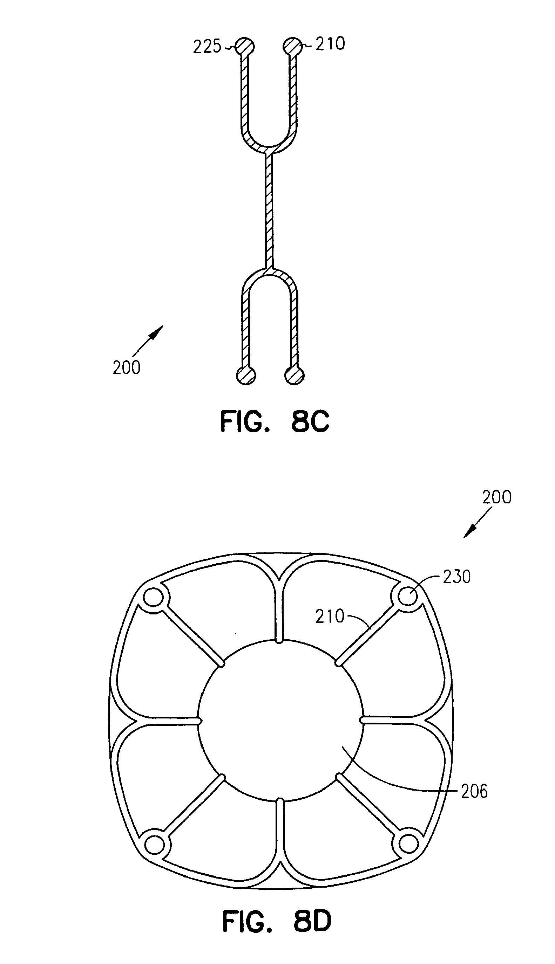

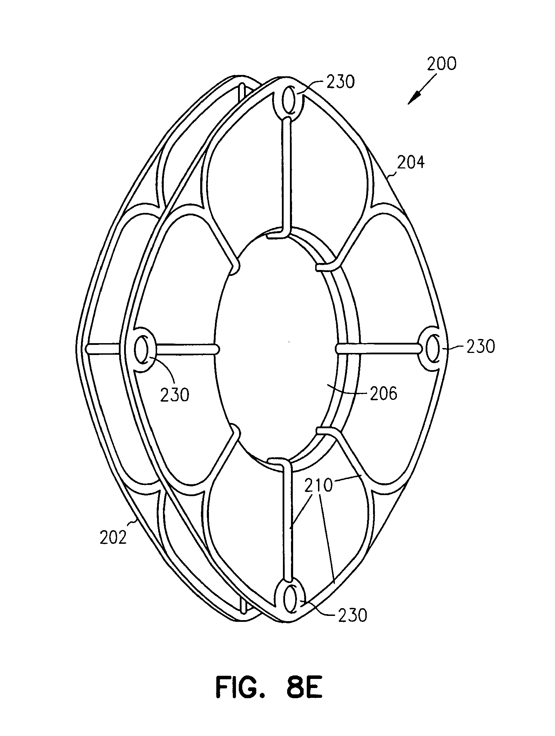

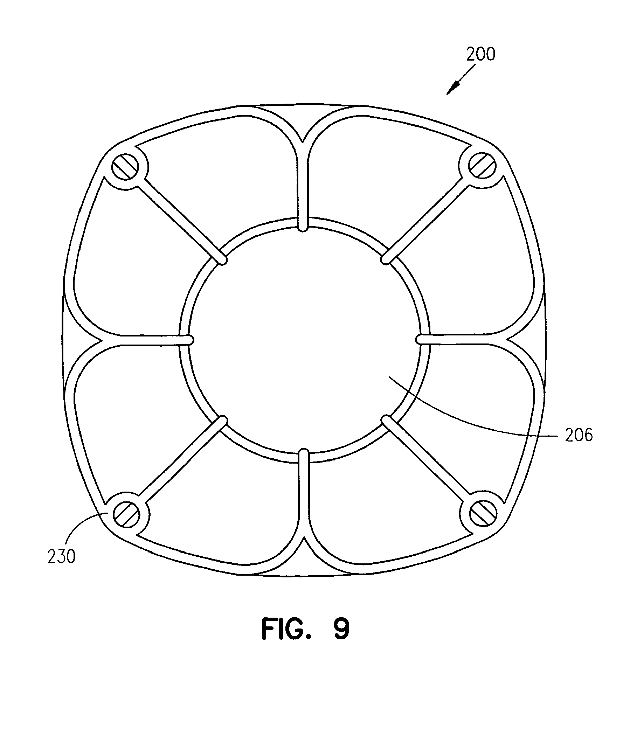

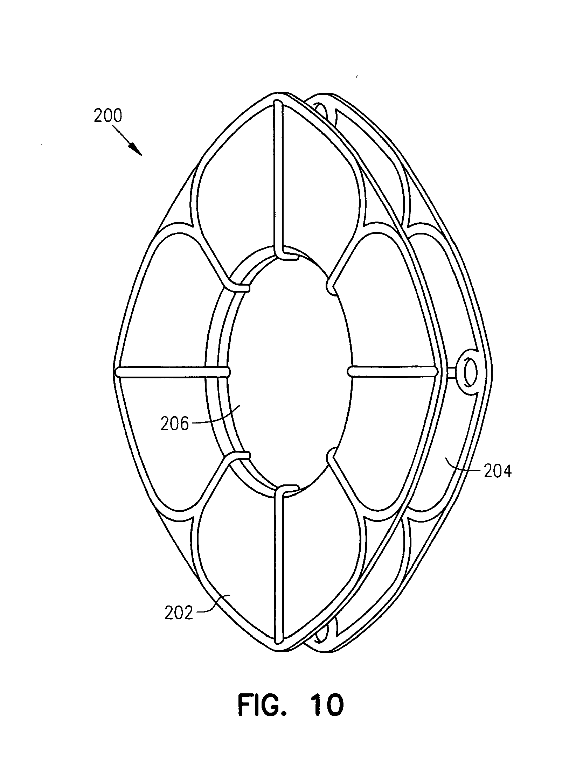

Figures 8A-10 show a vascular occlusion device 200 according to one embodiment not forming part of the present invention. Device 200 includes a pair of disk members defining a pair of flange members 202 and 204 connected by a central area 206. The skeleton structure 210 of occlusion device 200 includes an appearance of the petals of a flower. The skeleton 210 of device 200 runs across from one side to the other through a central area 206. In this example, skeleton 210 includes rib portions 225 running towards and around the periphery of each flange of the device.

Holes 230 are provided similar to holes 130 described above. Again, the central area 206 can be dimensioned to provide a self-centering device by having an outer rim surface 220 to abut the defect. While the disk shape shown is square- shaped, it may be round, oval, rectangular, or any other desirable shape. The thickness of the skeleton is variable and is determined by the occlusive pressures that will force the device from the defect. The thickness of the membrane can be variable. In one example, the membrane portion can be about 0,254 mm (0.010 inches) or less, with the ribs being about 0,502 mm (0.020 inches) to 0,635 mm (0.025 inches). The membrane in both the flanges can be perforated, and sieve like, to reduce the bulk of the device, so that it can be more easily loaded into a catheter. It would be desirable for the central area to be free of perforations for the shunt to be occluded. In one embodiment, the central area 206 is about 10 mm in diameter and each flange has a diameter of approximately 28 mm.

Device 200 can be constructed of the same material as for device 100 and the above discussion is incorporated herein by reference.

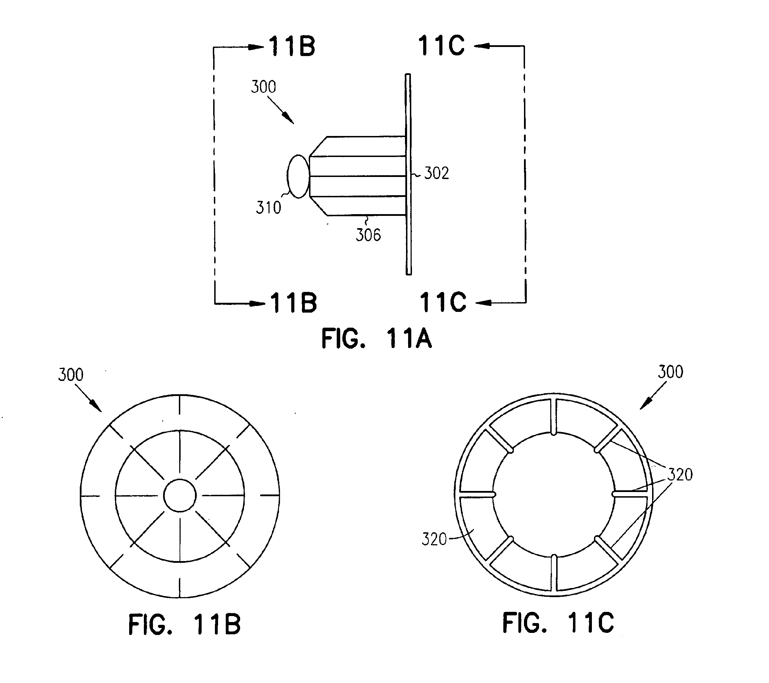

Figures 11A-11C show a device 300 for closure of patent ductus arteriosii (PDA), according to one embodiment not forming part of the present invention. Device 300 includes only one disk 302. There is a central tubular area 306 that is sized to fit into the patent ductus arteriosus. The circular ring 310 is for the release mechanism. Device 300 can be loaded into a catheter and this is used to cross the PDA. The circular disk 302 and the tubular section 306 are opened in the aorta and then it is pulled back into the PDA, so that the disk occludes the defect, and the tubular section seated in the PDA, keeps it in place and centered. If required, the device can be drawn back into the catheter for retrieval in case of sub-optimal deployment.

Device 300 can be constructed of the same material as for device 100 and the above discussion is incorporated herein by reference. Device 300 can include ribs 320 extending outwardly along a face of the disk 302. Again, in one embodiment, device 300 can be formed as an integral, one-piece design. For example, device 300 can be molded from a plastic material, such as a bioabsorbable material as discussed above.

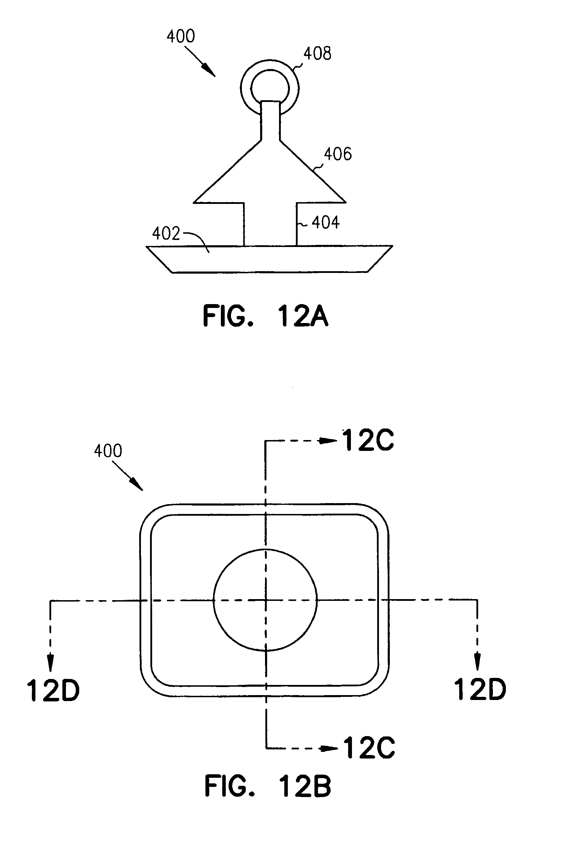

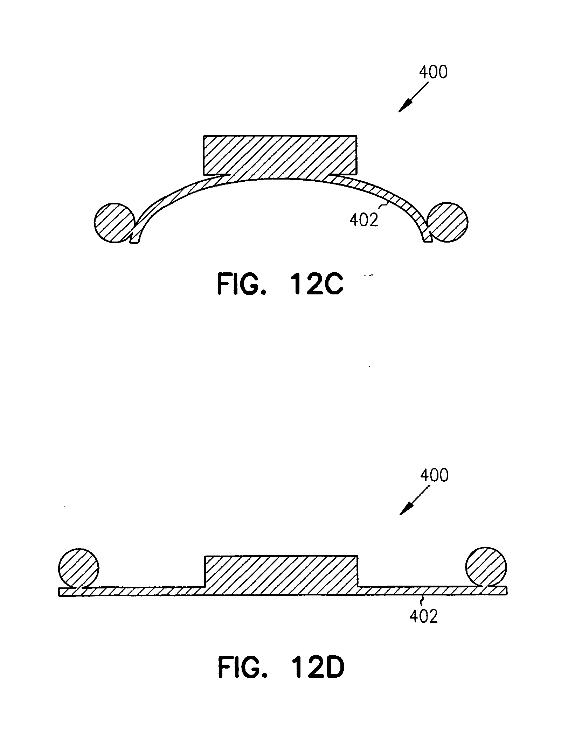

Figures 12A-13D show a device 400 that can be used to occlude puncture sites in the fomoral artery or vein after percutaneous interventions, in accordance with one embodiment not forming part of the present invention. In one example, device 400 includes a bio-adsorbable polymer. It has an intra-vascular member 402 that is rectangular in shape or oval. This is connected by a wide "waist" 404 to the extra-vascular member 406, that leads to a ring 408. One feature of this device is that the waist is designed to be slightly larger than the puncture hole in the artery or vein. For instance, with 6, 8 or 10 French catheter systems, the hole in the vessel can be as large as 1.98, 2.64, or 3.3 mm. The occluder is designed in different sizes so that the waist between the intravascular and extravascular disks are 2,2. 75 or 3.5 mm in diameter. This allows the present device to fit into the hole and tightly occlude it.

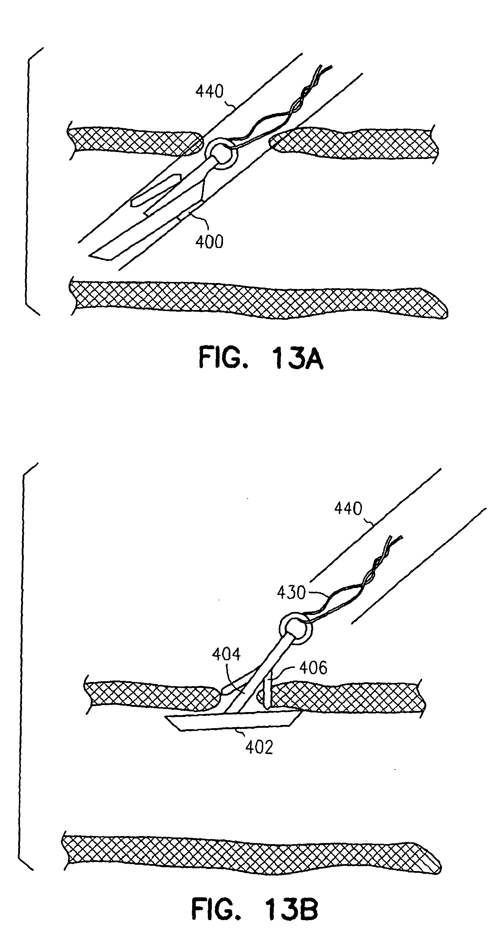

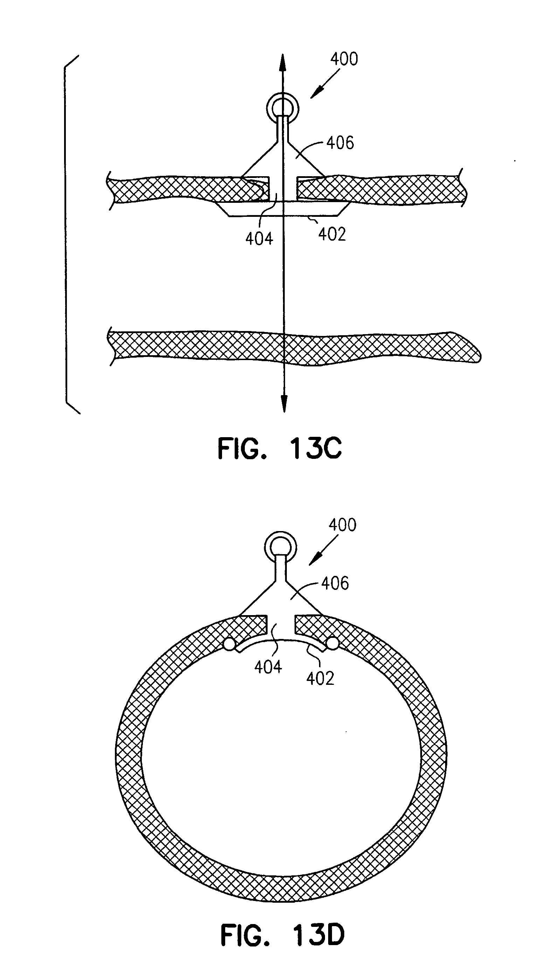

Figures 12B-12D show the shape of intra-vascular member 402 according to one embodiment. In this example, member 402 has a rectangle shape of approximately 6 mm by 8 mm, and which is curved to form a partial cylindrical shape. This allows the member to sit flush against an inner surface along the axis of the artery (See Figure 13D).

The external disk 406 is structured to prevent device 400 from prolapsing back into the artery and producing a total occlusion of the artery. Device 400 can be constructed of the same material as for device 100 and the above discussion is incorporated herein by reference.

Figures 13A-13D show one example of device 400 in use. For example, after a percutaneous procedure, an angiogram is obtained to identify the site of puncture, to ensure that it is above the bifurcation of the femoral artery. Device 400 is loaded into its sheath and this is passed through the previous sheath into the common iliac artery. The device is partially extruded until the intravascular disk 402 opens inside the artery. The whole unit is withdrawn, until the intravascular disk is snug against the artery. The device is held in place, using the release device 430, and the sheath 440 is drawn back so that the extravascular disk is opened. The sheaths are drawn back. The "self-centering" mechanism of the device permits the device to tightly occlude the defect. The intravascular disk prevents the device from being blown out by the intra-arterial pressure. Once hemostasis is confirmed, the device is released. It is anticipated that the bioabsorbable polymer would resorb over the course of a few weeks and heal with no foreign bodies remaining in the site.

Moreover, in one or more embodiments discussed above, the material of a device can be modified as necessary to prolong or shorten the resorption period to allow for predictable resorption times.

It is understood that the above description is intended to be illustrative. The scope of the invention should, therefore, be determined with reference to the appended claims,

## Claims

1. An occlusion device (100, 200) comprising:
first and second disk members (102, 104), each of the first and second disk members (102, 104) having a self-expanding, non-metallic structure including a plurality of integral rib members (125) which are flexible so as to be compressible within a catheter and are biased towards an open position to expand the disk members (102, 104) when removed from the catheter, and
a central rim (120) which is collapsible and by means of which the first and second disk members (102, 104) are connected together, and wherein the occlusion device (100) includes a sinuous structure around the central rim (120).

2. The occlusion device of claim 1, wherein the occlusion device (100, 200) includes a bioabsorbable material.

3. The occlusion device of claim 2, wherein the bioabsorbable material includes polyglycolic acid.

4. The occlusion device of claim 2, wherein the bioabsorbable material includes polydioxanone.

5. The occlusion device of claim 2, wherein the bioabsorbable material includes polylactic acid.

6. The occlusion device of claim 2, wherein the bioabsorbable material includes polycaprolactone.

7. The occlusion device of claim 1, wherein the first and second disk members (102, 104) are connected together at a central area (106) having a periphery and a plurality of ribs (125) are located around the periphery of the central area (106).

8. The occlusion device of claim 1, wherein each of the plurality of ribs (125) are radially oriented on the first and second disk members (102, 104).

9. The occlusion device of claim 1, wherein at least a portion of the plurality of ribs (125) define a petal-shaped configuration.

10. The occlusion device of claim 1, wherein the first and second disk members (102, 104) have a central area (106) therebetween, the central (106) being dimensioned to extend across a hole in a tissue, wherein the central portion (106) includes an outer periphery having a plurality of ribs to bias the periphery against the tissue.

11. The occlusion device of claim 1, further including one or more holes (130) formed in one of the disk members (102, 104) proximate a periphery of the disk member.

12. The occlusion device of claim 11, wherein each of the one or more holes are encircled by a portion of the rib members (125).

13. The occlusion device of claim 1, wherein one or more of the rib members (125) are thinner towards an outer end of the rib member.

14. The occlusion device of claim 1, wherein the occlusion device (100, 200) is a one-piece integral device.

15. The occlusion device of claim 1, wherein the device includes a single integrated molded unit including the first and second disk members, the ribs and a centering central disk.

16. The occlusion device of claim 1, wherein the device includes a single integrated molded unit formed of a bioabsorbable material, including the first and second disk members, the ribs and a centering central disk, the central disk being compressible and dimensioned to extend across a hole in a tissue when expanded.

## Patentansprüche

1. Verschlussvorrichtung (100, 200), aufweisend:
ein erstes und ein zweites Scheibenelement (102, 104), wobei das erste und das zweite Scheibenelement (102, 104) jeweils einen selbst aufspreizenden, nicht metallischen Aufbau haben, der mehrere integrale Rippenelemente (125) aufweist, die biegsam sind, so dass sie innerhalb eines Katheters zusammengedrückt werden können, und die zu einer offenen Position zum Aufspreizen der Scheibenelemente (102, 104), wenn sie von dem Katheter entfernt werden, vorgespannt sind, und
einen zentralen Rand (120), der zusammenlegbar ist und mittels welchem das erste und das zweite Scheibenelement (102, 104) miteinander verbunden sind, und wobei die Verschlussvorrichtung (100) eine wellenförmige Struktur um den zentralen Rand (120) aufweist.

2. Verschlussvorrichtung nach Anspruch 1, wobei die Verschlussvorrichtung (100, 200) ein bioabsorbierbares Material aufweist.

3. Verschlussvorrichtung nach Anspruch 2, wobei das bioabsorbierbare Material Polyglykolsäure aufweist.

4. Verschlussvorrichtung nach Anspruch 2, wobei das bioabsorbierbare Material Polydioxanon aufweist.

5. Verschlussvorrichtung nach Anspruch 2, wobei das bioabsorbierbare Material Polymilchsäure aufweist.

6. Verschlussvorrichtung nach Anspruch 2, wobei das bioabsorbierbare Material Polycaprolacton aufweist.

7. Verschlussvorrichtung nach Anspruch 1, wobei das erste und das zweite Scheibenelement (102, 104) miteinander an einem zentralen Bereich (106) verbunden sind, der einen Umfang hat und wobei mehrere Rippen (125) um den Umfang des zentralen Bereichs (106) angeordnet sind.

8. Verschlussvorrichtung nach Anspruch 1, wobei jede der mehreren Rippen (125) radial auf dem ersten und dem zweiten Scheibenelement (102, 104) ausgerichtet ist.

9. Verschlussvorrichtung nach Anspruch 1, wobei mindestens ein Teil der mehreren Rippen (125) eine blütenblattförmige Konfiguration definiert.

10. Verschlussvorrichtung nach Anspruch 1, wobei das erste und das zweite Scheibenelement (102, 104) zwischeneinander einen zentralen Bereich (106) haben, wobei der zentrale Bereich (106) bemessen ist, um sich über ein Loch in einem Gewebe zu erstrecken, wobei der zentrale Bereich (106) einen Außenumfang aufweist, der mehrere Rippen aufweist, um den Umfang gegen das Gewebe vorzuspannen.

11. Verschlussvorrichtung nach Anspruch 1, die ferner ein oder mehrere Löcher (130) aufweist, die in einem der Scheibenelemente (102, 104) in der Nähe eines Umfangs des Scheibenelements ausgebildet ist/sind.

12. Verschlussvorrichtung nach Anspruch 11, wobei das eine oder die mehreren Löcher von einem Teil der Rippenelemente (125) eingefasst ist.

13. Verschlussvorrichtung nach Anspruch 1, wobei eines oder mehrere Rippenelemente (125) zu einem Außenende des Rippenelements dünner ausgebildet sind.

14. Verschlussvorrichtung nach Anspruch 1, wobei die Verschlussvorrichtung (100, 200) eine einstückige Vorrichtung ist.

15. Verschlussvorrichtung nach Anspruch 1, wobei die Vorrichtung eine einzige integrierte Formeinheit aufweist, die das erste und das zweite Scheibenelement, die Rippen und eine zentrierende Zentralscheibe aufweist.

16. Verschlussvorrichtung nach Anspruch 1, wobei die Vorrichtung eine einzige integrierte geformte Einheit aufweist, die aus einem bioabsorbierbaren Material gebildet ist, die das erste und das zweite Scheibenelement, die Rippen und eine zentrierende Zentralscheibe aufweist, wobei die Zentralscheibe zusammendrückbar und bemessen ist, um sich bei ihrem Aufspreizen über ein Loch in einem Gewebe zu erstrecken.

## Revendications

1. Dispositif d'occlusion (100, 200) comportant :
des premier et second éléments formant disques (102, 104), chacun des premier et second éléments formant disques (102, 104) ayant une structure non métallique auto-expansible incluant une pluralité d'éléments formant nervures d'un seul tenant (125) qui sont souples de manière à pouvoir être comprimés à l'intérieur d'un cathéter et qui sont rappelés vers une position ouverte pour détendre les éléments formant disques (102, 104) lorsqu'ils sont retirés du cathéter, et
une couronne centrale (120) qui peut être aplatie et au moyen de laquelle les premier et second éléments formant disques (102, 104) sont reliés l'un à l'autre, et
le dispositif d'occlusion (100) incluant une structure sinueuse autour de la couronne centrale (120).

2. Dispositif d'occlusion selon la revendication 1, le dispositif d'occlusion (100, 200) incluant un matériau bioabsorbable.

3. Dispositif d'occlusion selon la revendication 2, dans lequel le matériau bioabsorbable inclut de l'acide polyglycolique.

4. Dispositif d'occlusion selon la revendication 2, dans lequel le matériau bioabsorbable inclut du polydioxanone.

5. Dispositif d'occlusion selon la revendication 2, dans lequel le matériau bioabsorbable inclut de l'acide polylactique.

6. Dispositif d'occlusion selon la revendication 2, dans lequel le matériau bioabsorbable inclut de la polycaprolactone.

7. Dispositif d'occlusion selon la revendication 1, dans lequel les premier et second éléments formant disques (102, 104) sont reliés l'un à l'autre au niveau d'une zone centrale (106) ayant une périphérie, et une pluralité de nervures (125) est située autour de la périphérie de la zone centrale (106).

8. Dispositif d'occlusion selon la revendication 1, dans lequel chacune nervure parmi la pluralité de nervures (125) est orientée radialement sur les premier et second éléments formant disques (102, 104).

9. Dispositif d'occlusion selon la revendication 1, dans lequel au moins une partie de la pluralité de nervures (125) définit une configuration en forme de corolle.

10. Dispositif d'occlusion selon la revendication 1, dans lequel les premier et second éléments formant disques (102, 104) ont une zone centrale (106) entre eux, la zone centrale (106) étant dimensionnée pour s'étendre à travers un trou dans un tissu, dans lequel la zone centrale (106) inclut une périphérie extérieure ayant une pluralité de nervures pour rappeler la périphérie contre le tissu.

11. Dispositif d'occlusion selon la revendication 1, comportant également un ou plusieurs trous (130) formés dans l'un desdits éléments formant disques (102, 104) à proximité d'une périphérie de l'élément formant disque.

12. Dispositif d'occlusion selon la revendication 11, dans lequel le ou les trous sont chacun encerclés par une partie des éléments formant nervures (125).

13. Dispositif d'occlusion selon la revendication 1, dans lequel un ou plusieurs des éléments formant nervures (125) sont amincis vers une extrémité extérieure de l'élément formant nervure.

14. Dispositif d'occlusion selon la revendication 1, le dispositif d'occlusion (100, 200) étant un dispositif monobloc.

15. Dispositif d'occlusion selon la revendication 1, le dispositif incluant une seule unité moulée intégrée comportant les premier et second éléments formant disques, les nervures et un disque central de centrage.

16. Dispositif d'occlusion selon la revendication 1, le dispositif incluant une seule unité moulée intégrée formée d'un matériau bioabsorbable, comportant les premier et second éléments formant disques, les nervures et un disque central de centrage, le disque central pouvant être comprimé et étant dimensionné pour s'étendre à travers un trou dans un tissu lorsqu'il est détendu.
